# FASCICULE DE BREVET EUROPEEN

(11) **EP 3 031 491 B1**
(45) Date de publication et mention de la délivrance du brevet: **30.01.2019**
(21) Numéro de dépôt: 15197790.7
(22) Date de dépôt: 03.12.2015
(51) Int. Cl.: A61N 1/36, A61H 1/00, A61B 5/00, A61H 23/00

(54) **SYSTÈME DE TRAITEMENT D'UN TROUBLE RESPIRATOIRE PAR STIMULATION KINESTHÉSIQUE, AVEC SÉLECTION DES STRATÉGIES DE STIMULATION**
SYSTEM ZUR BEHANDLUNG VON ATEMBESCHWERDEN DURCH KINÄSTHETISCHE STIMULATION MIT AUSWAHL DER STIMULATIONSSTRATEGIEN
SYSTEM FOR TREATING A RESPIRATORY CONDITION BY KINESTHETIC STIMULATION, WITH SELECTION OF STIMULATION STRATEGIES

(30) Priorité: 08.12.2014 FR 1462039
(43) Date de publication de la demande: 15.06.2016
(73) Titulaire: Sorin CRM SAS, 92140 Clamart Cedex (FR); INSTITUT NATIONAL DE LA SANTE ET DE LA RECHERCHE MEDICALE (INSERM), 75013 Paris (FR); Université Grenoble Alpes, 38400 Saint-Martin-d'Hères (FR); Université de Rennes 1, 35065 Rennes Cedex (FR)
(72) Inventeur: GRAINDORGE, Laurence, 44470 Thouaré sur Loire (FR); AMBLARD, Amel, 92330 Sceaux (FR); FEUERSTEIN, Delphine, 92130 Issy les Moulineaux (FR); HERNANDEZ, Alfredo, 35510 Cesson Sévigné (FR); PEPIN, Jean-Louis, 38000 Grenoble (FR)
(74) Mandataire: Grünecker Patent- und Rechtsanwälte PartG mbB

(56) Documents cités:
- EP-A1- 1 317 943
- WO-A1-2007/141345
- US-A1- 2010 048 985
- US-A1- 2014 051 938

## Description

L'invention concerne le diagnostic et le traitement des troubles du sommeil.

Elle concerne plus particulièrement l'utilisation à cet effet d'un dispositif dit de "stimulation kinesthésique", c'est-à-dire d'un dispositif de stimulation sensorielle externe du patient au moyen d'un vibreur en contact avec la peau dans une région sensible, précise du corps de ce patient. L'activation de ce vibreur a pour effet d'exciter localement les terminaisons cutanées ou mécanorécepteurs de la peau, et de déclencher une réponse du système nerveux autonome du patient, à prépondérance sympathique (ci-après "réponse autonomique").

La réponse autonomique à une activation sympathique est observable sur les principaux effets modulateurs de l'activité cardiaque, par exemple un effet chronotrope (augmentation de la fréquence cardiaque, ou diminution des intervalles RR) et un effet inotrope (augmentation de la contractilité cardiaque).

Cette réponse autonomique est également observable sur la vasoconstriction périphérique, qui est augmentée en cas d'activation autonomique sympathique.

Outre ces conséquences sur l'activité cardiaque, une activation sympathique provoque des réponses sur le système respiratoire ou sur le système nerveux central (réveils autonomiques).

Il s'agit d'un procédé non invasif, qui permet d'agir sur un certain nombre de troubles du sommeil en alternative aux approches thérapeutiques classiques basées sur l'application d'une pression positive continue à travers un masque facial (traitement dit par CPAP), sur l'utilisation d'une orthèse de propulsion mandibulaire et/ou sur la stimulation électrique du nerf grand hypoglosse, qui implique la pose d'un implant de type stimulateur. En particulier, la pathologie respiratoire connue sous le nom de "syndrome d'apnée du sommeil" (SAS) est caractérisée par la survenue fréquente (au moins 10 à 20 fois par heure) d'apnées pendant une phase de sommeil du patient, une "apnée" (ou pause respiratoire) étant définie comme un arrêt temporaire de la fonction respiratoire de durée supérieure à 10 s. Elle peut également être caractérisée par la survenue d'hypopnées dans les mêmes conditions, une "hypopnée" étant définie comme une décroissance importante (mais sans interruption) du débit respiratoire, typiquement une décroissance de plus de 50 % par rapport à une moyenne de référence antérieure.

Cette pathologie atteint plus de 4 % de la population et plus de 50 % des patients souffrant d'insuffisance cardiaque. Afin de protéger l'individu contre l'asphyxie liée à la diminution de la concentration en oxygène du sang pendant l'interruption ou la réduction du débit respiratoire, le corps s'adapte mais avec un effet délétère sur le sommeil, entraînant des micro-réveils inconscients. Il s'ensuit en phase d'éveil une somnolence diurne avec perte d'attention et un accroissement des risques d'accident de la route. Par ailleurs, plusieurs études ont montré chez les patients souffrant de SAS une plus grande incidence de troubles tels qu'hypertension artérielle, arythmies ventriculaires, infarctus myocardique et insuffisance cardiaque.

Plusieurs documents décrivent la possibilité de stopper les épisodes d'apnée grâce à un traitement par stimulation.

Ainsi, le US 5 555 891 A décrit un système de stimulation vibrotactile pour arrêter les apnées chez les nouveau-nés. L'objectif est de fournir un système capable de détecter les apnées et de stimuler l'enfant pour arrêter l'apnée, avec une énergie de stimulation qui peut varier pour éviter l'habituation. L'énergie appliquée est importante et impliquera souvent un réveil.

Le WO 2007/141345 A1 décrit un système de *télémonitoring* pour les services de néonatologie, permettant de détecter et de stimuler les apnées-bradycardies du nourrisson. Cette demande mentionne un ajustement de l'énergie de stimulation en fonction de la fréquence cardiaque mesurée du nourrisson. Dans la méthode proposée aucune analyse des signaux respiratoires n'est réalisée. Ceci empêche la différentiation des types de trouble de sommeil détectés ou attendus, et donc, l'ajustement des stratégies de stimulation en fonction dudit type de trouble. De plus, cette approche limite la délivrance de la thérapie à l'intervalle de temps pendant lequel le trouble est présent.

Le WO 96/28093 A1 enseigne également un système qui délivre un stimulus pour réduire la fréquence ou la durée d'un épisode d'apnée. Au niveau de la stimulation, ce document décrit simplement des limites basse et haute de l'énergie de stimulation pour espérer arrêter l'apnée sans réveiller le patient.

Le US 2008/009915 A1 décrit un système qui détecte les troubles respiratoires à l'aide d'une canule nasale ou autre et qui applique une stimulation notamment vibratoire dans la région de l'oreille. L'objectif est d'arrêter l'apnée, sans réveiller le patient, par une stimulation qui peut-être modulée, manuellement ou automatiquement, en fonction de caractéristiques physiologiques du patient ou des cycles du sommeil. Ce document cite également d'une façon générale l'optimisation des paramètres de stimulation pour s'adapter à la gravité des troubles du patient, sans toutefois donner de détail sur leur ajustement. La variation des paramètres pour éviter l'habituation est également citée.

Le US 2010/0048985 A1 décrit un dispositif comparable, permettant d'appliquer des stimuli par des moyens de natures diverses (stimulation sonore ou ultrasonore de l'oreille, stimulation oculaire, agitateur mécanique, etc.). Le dispositif comprend également des moyens d'analyse de l'activité respiratoire permettant d'évaluer l'efficacité de la stimulation afin que le patient ou le médecin puissent modifier si besoin le paramétrage du générateur avec un dosage différent des stimuli.

Le US 2008/0154330 A1 décrit un système de stimulation électrique du diaphragme pour stabiliser la respiration. La thérapie est déclenchée quand une instabilité respiratoire est détectée, et arrêtée lorsqu'un nombre prédéterminé de cycles stables ont été détectés. Aucune indication n'est donnée sur une optimisation éventuelle de cette thérapie, basée sur une analyse de stabilité de la respiration. De plus, ce traitement par stimulation du diaphragme n'est pas applicable aux apnées ou hypopnées d'origine obstructive.

Enfin, le WO 2009/154458 A2 enseigne un système qui détecte les apnées et en retour provoque un réflexe d'inspiration par stimulation dans la région de l'oreille. Divers moyens de détection des apnées sont cités. La stimulation peut-être électrique ou mécanique. La stratégie de stimulation est minimale, elle consiste à appliquer des trains de stimulations aussi longtemps que le trouble est présent. Les paramètres de stimulation sont indiqués comme pouvant varier, sans qu'il soit toutefois proposé de règles de variation. Une variation aléatoire des paramètres est également citée pour éviter l'habituation. Mais ce document reste très vague sur les paramètres de stimulation. Il propose seulement, de façon très générale :
- une stimulation par voie mécanique, électrique ou acoustique ;
- une fréquence de stimulation variant entre 1 et 500 Hz ; et
- une durée de stimulation et un temps d'attente entre deux stimulations variant entre 0,5 et 10 s.

Il est évident que la stimulation sera plus ou moins efficace en fonction des valeurs choisies, les éléments du tronc cérébral ayant par exemple une sensibilité qui varie très largement avec la gamme de fréquences. Par ailleurs, en faisant par exemple varier l'énergie de stimulation de manière aléatoire, on risque de diminuer l'efficacité du traitement (si l'énergie est trop faible) ou de réveiller le patient (si l'énergie est trop importante). Enfin, dans ce document, le traitement s'arrête dès la reprise respiratoire, c'est-à-dire dès la détection du réflexe inspiratoire que l'on cherche à déclencher.

Ainsi l'état de l'art ne donne que très peu d'enseignements sur la façon précise dont la stimulation doit être délivrée. Or une stimulation mal ajustée ou aléatoire peut entrainer :
- une inefficacité, si la stimulation n'est pas adéquate et adaptée à la réponse que l'on veut provoquer ;
- une accoutumance à court ou moyen terme entrainant une inefficacité du traitement, si celui-ci est délivré trop souvent ou à mauvais escient ;
- enfin, un réveil du patient et donc une déstructuration du sommeil, ce qui est précisément ce que l'on veut éviter en traitant les apnées.

Un *premier aspect* de la présente invention repose sur la découverte par les inventeurs de ce qu'un ajustement fin des paramètres de stimulation peut avoir un impact important sur la qualité et l'efficacité du traitement. Un *second aspect* de l'invention repose sur la découverte de ce qu'un contrôle temporel fin de la période de stimulation, et tout particulièrement du moment de son arrêt, peut avoir un impact important sur la qualité et l'efficacité du traitement.

Plus précisément, l'invention propose un dispositif de traitement d'un trouble respiratoire chez un patient par stimulation kinesthésique, comprenant de manière en elle-même connue, notamment d'après les US 2010/0048985 A1 et WO 96/28093 A1 précités :
- des moyens de commande aptes à produire des signaux de commande de stimulation kinesthésique en réponse à la détection d'un trouble respiratoire ; et
- au moins un effecteur kinesthésique apte à être appliqué sur un site cutané externe du patient, et comprenant un transducteur électromécanique vibrant apte à recevoir les signaux de commande et à délivrer une énergie de stimulation kinesthésique déterminée par lesdits signaux de commande.

Selon le *premier aspect* précité, le dispositif est caractérisé en ce qu'il comprend en outre :
- des moyens de détermination automatique de traitement, aptes à sélectionner dynamiquement, en fonction d'un type de trouble attendu ou détecté, une stratégie de stimulation parmi un ensemble de stratégies de simulation mémorisées.

Selon diverses caractéristiques subsidiaires avantageuses :
- les stratégies de stimulation comprennent au moins une stratégie de stimulation par succession d'impulsions de stimulation d'un premier type, et au moins une stratégie de stimulation par succession d'impulsions de stimulation d'un second type, les impulsions du second type ayant une durée d'impulsion supérieure à celle des impulsions du premier type et/ou les impulsions du second type étant espacées entre elles d'un intervalle de durée supérieure à celle des impulsions du premier type ;
- les impulsions du premier type ont une durée d'impulsion comprise entre 2 et 3 secondes et sont espacées entre elles d'un intervalle de durée comprise entre 2 et 3 secondes, et les impulsions du second type ont une durée d'impulsion supérieure à celle des impulsions du premier type et sont espacées entre elles d'un intervalle de durée supérieure à celle des impulsions du premier type ;
- les moyens de détermination automatique de traitement sont aptes à plafonner la valeur de l'énergie de stimulation à une valeur maximale ;
- les moyens de détermination automatique de traitement sont aptes à sélectionner, en fonction du type de trouble attendu ou détecté, soit une stratégie de stimulation à énergie élevée et thérapie précoce, soit une stratégie de stimulation à énergie moindre et thérapie plus tardive ;
- les moyens de détermination automatique de traitement sont aptes à relever de façon incrémentale le niveau d'énergie à partir de la valeur initiale, aussi longtemps qu'un trouble respiratoire intervenu ne disparait pas ;
- les moyens de détermination automatique de traitement sont aptes à comparer chaque valeur d'énergie incrémentée à une valeur maximale ;
- les moyens de détermination automatique de traitement sont aptes à forcer le niveau d'énergie à une valeur supérieure à la valeur maximale admissible, en cas de détection d'un épisode grave de trouble respiratoire.

Le *second aspect* précité vise un dispositif comprenant, de manière en elle-même connue :
- des moyens de commande aptes à produire des signaux de commande de stimulation kinesthésique en réponse à la détection d'un trouble respiratoire ;
- au moins un effecteur kinesthésique apte à être appliqué sur un site cutané externe du patient, et comprenant un transducteur électromécanique vibrant apte à recevoir les signaux de commande et à délivrer une énergie de stimulation kinesthésique déterminée par lesdits signaux de commande ; et
- des moyens pour déterminer l'efficacité d'une stimulation, aptes à détecter la disparition dudit trouble.

De façon caractéristique de ce second aspect, le dispositif comprend en outre :
- des moyens pour prolonger la commande de stimulation pendant une durée de stabilisation déterminée suivant la disparition du trouble.

Selon diverses caractéristiques subsidiaires avantageuses :
- les moyens pour prolonger la commande de stimulation sont aptes à appliquer après la disparition du trouble des impulsions ayant même durée et même espacement que celles qui ont causé la disparition du trouble ;
- les moyens pour prolonger la commande de stimulation sont aptes à déterminer la durée de prolongation de la commande de stimulation à partir d'un nombre de cycles de respiration consécutifs à la reprise de la respiration lors de la disparition du trouble ;
- le système comprend en outre des moyens pour surveiller l'apparition d'un nouvel épisode de trouble respiratoire pendant une durée déterminée suivant la fin d'un épisode de trouble ;
- le système comprend en outre des moyens pour modifier la durée de prolongation de la commande de stimulation, en fonction de l'apparition ou non d'un nouvel épisode de trouble respiratoire pendant ladite durée de stabilisation déterminée ;
- les moyens pour prolonger la commande de stimulation sont aptes à ajuster dynamiquement la durée de prolongation de la commande de stimulation par un apprentissage fonction des récidives éventuelles observées au cours du temps ;
- les moyens de commande sont en outre aptes à limiter la délivrance de l'énergie de stimulation kinesthésique à un nombre prédéterminé d'impulsions de stimulation successivement délivrées ;
- le nombre prédéterminé d'impulsions peut prendre deux valeurs différentes, sélectionnées par les moyens de commande en fonction du type de trouble respiratoire.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 illustre schématiquement un système selon l'invention, posé sur un patient.
Les Figures 2 et 3 sont des chronogrammes illustrant des impulsions de stimulation appliquées à un patient, avec une modélisation des variations résultantes de l'équilibre sympathovagal, respectivement pour deux durées d'impulsions différentes.
La Figure 4 illustre schématiquement un processus de sélection de l'un parmi deux jeux de paramètres de stimulation en fonction du type de trouble observé.
La Figure 5 est un organigramme plus détaillé des différentes étapes du processus de sélection et d'application de stimulations avec des paramètres différents en fonction du type de trouble.
La Figure 6 est un chronogramme illustrant l'arrêt de la stimulation après disparition du trouble selon l'art antérieur.
La Figure 7 est un chronogramme illustrant l'arrêt de la stimulation après disparition du trouble, selon un aspect de l'invention.
La Figure 8 est un organigramme détaillé d'un processus d'ajustement du moment d'arrêt de la stimulation selon l'aspect précité.
La Figure 9 illustre sur un diagramme temporel l'agencement des différentes fonctions de pilotage de la stimulation selon les enseignements de l'invention.

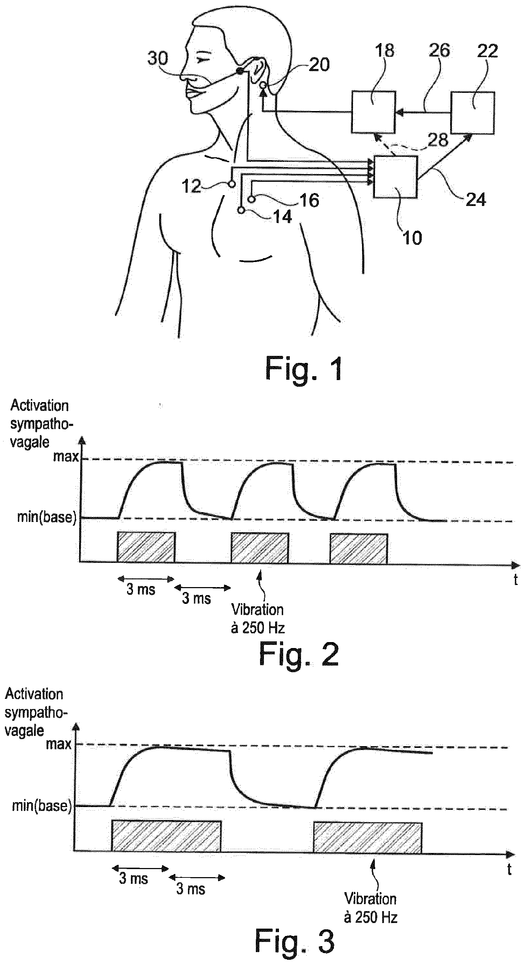

La Figure 1 illustre schématiquement les principaux composants d'un système utilisable pour la mise en oeuvre de la présente invention.

Ce système comporte un enregistreur Holter 10 relié à divers capteurs ou électrodes 12, 14, 16, permettant de mesurer des signaux physiologiques tels que la fréquence cardiaque, la respiration, la saturation en oxygène, l'onde de pouls, le phonocardiogramme, etc. Dans la suite, on s'intéressera principalement à l'amplitude ou à la pression respiratoire, qui sont des paramètres simples à obtenir, mais cette mesure n'est pas limitative et l'invention peut être aussi bien mise en oeuvre à partir d'autres signaux physiologiques recueillis sur le patient.

Le système comprend par ailleurs un dispositif de stimulation kinesthésique, avec un boitier générateur 18 produisant des impulsions de commande d'un effecteur de stimulation kinesthésique 20, constitué par exemple d'un vibreur placé dans une région sensible de l'épiderme, typiquement (chez l'adulte) dans la région de l'os mastoïde au voisinage de l'oreille. La stimulation vibrotactile appliquée sur la peau par l'effecteur 20 est détectée par les récepteurs sensoriels ou mécanorécepteurs de l'organisme, et cette information est ensuite transmise par l'intermédiaire des nerfs sensitifs au système nerveux central autonome.

L'effecteur 20 est par exemple un transducteur du type *C10-100* de Preci-sion Microdrives ou *C2 Tactor* de Engineering Acoustics. Il s'agit d'un transducteur de quelques grammes susceptible d'émettre des vibrations grâce à un vibreur intégré excité par des trains d'impulsions d'amplitude et de durée variables, typiquement à une fréquence de 250 Hz qui est la fréquence nominale de résonance de cet effecteur particulier et qui est également la fréquence à laquelle les mécanorécepteurs de la peau sont les plus sensibles. D'autres types d'effecteur peuvent bien entendu être efficacement utilisés.

Le boitier de commande 18 est piloté par un microcontrôleur et possède des moyens permettant de régler l'intensité (c'est-à-dire l'énergie) de la stimulation kinesthésique, par variation contrôlée de l'amplitude et/ou du nombre, de la durée et/ou de la fréquence de stimulation des trains d'impulsions formant le signal appliqué à l'effecteur 20.

Le système comprend également un boitier 22 couplé au dispositif Holter 10 et au boitier de commande 18 par une liaison filaire ou sans fil respective 24, 26, de manière à recevoir des données du dispositif Holter 10, traiter ces données et produire en réponse des informations de contrôle de la stimulation kinesthésique qui seront transmises au boitier de commande 18. En variante, le traitement des données et le pilotage du boitier de commande 18 peuvent être opérés au sein même du dispositif Holter 10 et transmis directement par une liaison 28 au boitier 18.

Le système comprend enfin des moyens de mesure du débit respiratoire, par exemple une canule de pression nasale 30 (et/ou une canule buccale) ou autre type de capteur tel que thermistance ou capteur mécanique des variations de volume de l'abdomen et/ou de la cage thoracique (au moyen d'une ceinture équipée de capteurs sensibles à l'étirement). Ces moyens délivrent au dispositif Holter 10 un signal ventilatoire analysé en continu de manière à détecter en temps réel la survenue d'une apnée ou d'une hypopnée.

Le fonctionnement du système est le suivant : lorsqu'une apnée est détectée par le dispositif Holter 10, le boitier de commande 18 déclenche un stimulus kinesthésique propre à déclencher une réponse du système nerveux autonome de sorte que, en réponse, le système nerveux autonome va provoquer une variation de la respiration et donc enclencher un réflexe respiratoire qui endiguera l'apnée.

Le système selon l'invention est apte à déclencher des modes de stimulations différents, selon le type de trouble respiratoire.

A cet égard, les inventeurs ont imaginé d'adapter le mécanisme de la stimulation aux événements à traiter, en vue d'optimiser l'efficacité de la stimulation. Plus particulièrement, les apnées et hypopnées se répartissent en deux groupes principaux : les apnées centrales et les apnées obstructives. Ainsi, même si ces deux types d'événements peuvent d'une façon générale être arrêtés par une stimulation kinesthésique à l'aide du système décrit ci-dessus, le mécanisme opératoire de cette stimulation doit avantageusement être différencié pour être efficace.

On décrira en premier lieu le cas de l'apnée centrale.

Il a été observé que le mécanisme opératoire de l'arrêt des apnées/hypopnées centrales dans le système selon l'invention repose sur la stimulation du système neurovégétatif ou réflexe sympathovagal. Ce type de réponse s'obtient aisément de façon non invasive en stimulant les mécanorécepteurs de la peau. Des études ont démontré que pendant le sommeil, le système nerveux autonome reste réactif aux stimuli sensoriels externes et provoque une réponse physiologique. Ainsi l'application d'une stimulation sur la peau pendant le sommeil déclenche une réponse sympathovagale qui agit sur la fréquence cardiaque, la tension artérielle mais également sur la respiration.

Des expériences animales et des modélisations mathématiques de l'équilibre sympathovagal ont permis par ailleurs de déterminer les paramètres optimaux de stimulation de l'équilibre sympathovagal, sans toutefois le saturer.

Ainsi le chronogramme de la Figure 2 illustre une stimulation efficace de l'équilibre sympathovagal. La stimulation est effectuée par des salves im-pulsionnelles (ci-après "impulsions") pendant lesquelles est activée une vibration mécanique à la fréquence de 250 Hz, fréquence à laquelle les mécanorécepteurs de la peau sont les plus sensibles et donc optimale pour une stimulation kinesthésique.

La durée d'une telle impulsion correspond à l'atteinte du niveau maximum de stimulation de la balance sympathovagale. Ainsi, on est assuré de déclencher de façon efficace les réflexes dépendant de cette stimulation, dont l'augmentation de la respiration. Des expériences et modélisations ont permis d'estimer cette durée des impulsions à une valeur comprise entre environ 2 et 3 s.

Dès que ce niveau maximum est atteint, la stimulation est arrêtée afin de permettre au système sympathovagal de récupérer le plus rapidement possible. Lorsque l'équilibre sympathovagal est revenu à son niveau de base, minimal, alors la stimulation peut reprendre sans perdre de son efficacité et sans risquer une habituation à la stimulation. Les expériences et modélisations précitées ont permis d'estimer cet intervalle entre impulsions à une valeur comprise également entre 2 et 3 s.

La Figure 3 illustre la situation où les impulsions de stimulation sont trop longues : elles continuent alors que l'activation sympathovagale a atteint un plateau. Cette prolongation des impulsions n'augmente pas l'efficacité de stimulation car les réflexes ont été enclenchés de façon maximale au début du plateau. Par contre, elle ralentit la récupération du système qui a, en quelque sorte, saturé.

De plus, si la première stimulation n'a pas eu l'effet escompté, l'impulsion inutilement longue retarde la délivrance de la prochaine salve, et donc retarde d'autant la possibilité d'enrayer le trouble respiratoire.

Inversement, avec des impulsions de stimulation trop courtes, le risque est de ne pas stimuler efficacement l'équilibre sympathovagal et donc de ne pas provoquer le ou les réflexes attendus (typiquement l'augmentation de la fréquence cardiaque, de la tension ou de la respiration).

En résumé, les paramètres importants d'une stimulation kinesthésique pour les apnées/hypopnées centrales sont :
- une fréquence de vibration mécanique de 250 Hz ;
- une durée d'impulsion d'environ 2 à 3 s ;
- un intervalle entre impulsions (durée de récupération) compris entre environ 2 et 3 s.

Ces paramètres permettent d'optimiser la réponse sympathovagale, tout en évitant l'habituation et en permettant de réitérer le traitement aussi tôt que possible en cas d'inefficacité.

Si l'on considère maintenant le cas d'une apnée/hypopnée obstructive et non plus centrale, alors l'objectif de la stimulation kinesthésique dans l'apnée obstructive est de stimuler les nerfs des muscles commandant la respiration, notamment le nerf hypoglosse.

Le mécanisme expliqué précédemment pourra être efficace sur certains épisodes ou certaines personnes, car le déclenchement de la réponse sympathovagale va également exciter les fonctions nerveuses et donc le nerf hypoglosse, mais ce mécanisme pourrait ne pas être suffisant. Aussi, on a observé qu'il était souhaitable, contrairement au cas précédent, d'appliquer une stimulation continue, de 10 s ou davantage afin de stimuler le maximum de fibres nerveuses. La fréquence des vibrations de stimulation reste de préférence identique, aux alentours de 250 Hz : il s'agit là non plus de déclencher un réflexe mais de recruter le maximum de fibres nerveuses proches du point de stimulation.

Entre ces deux possibilités de thérapie, la sélection du mode de stimulation est, de façon caractéristique, opérée par le dispositif de façon automatique, en réponse à des moyens de différentiation en temps réel des épisodes centraux/obstructifs, et/ou en fonction de l'efficacité de l'une ou l'autre thérapie.

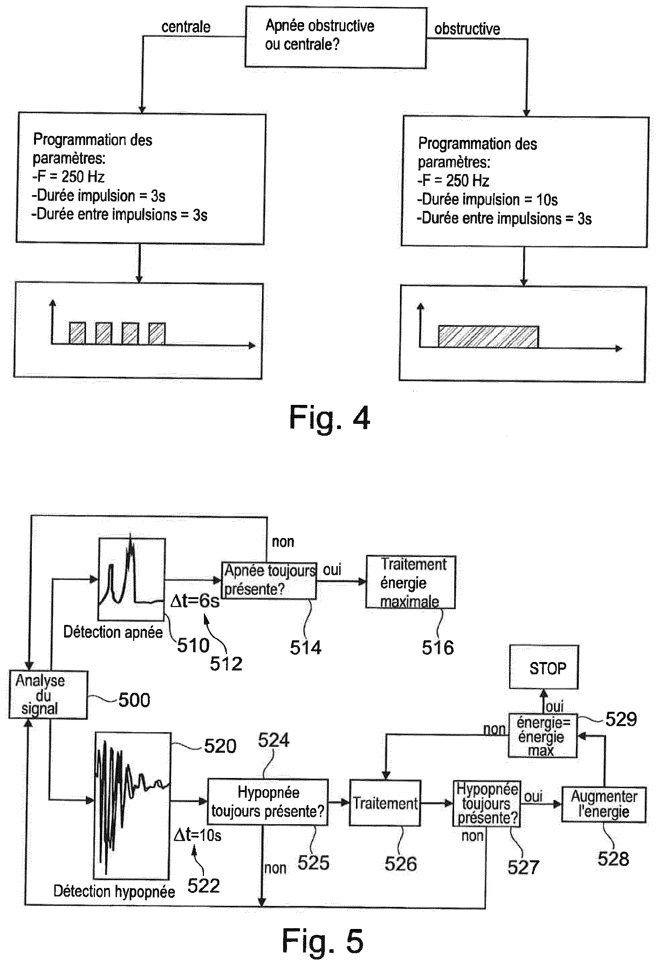

La Figure 4 illustre de façon synthétique le choix entre les deux stratégies de stimulation respectives, adaptées dans cet exemple aux deux types d'apnées.

Par ailleurs, le système de stimulation selon l'invention est apte à traiter les événements cliniques de façon différenciée.

Par exemple, une apnée est un épisode grave qui peut amener à une désaturation rapide. De plus, ce trouble, caractérisé par une absence de flux respiratoire, est facile à diagnostiquer avec une bonne sensibilité et spécificité. Enfin, il est important de stimuler au plus vite un épisode d'apnée car celui-ci sera réduit d'autant plus facilement qu'il est pris en charge rapidement.

Au contraire, une hypopnée est un phénomène clinique plus courant mais moins grave immédiatement. La détection de l'hypopnée est plus délicate et une bonne sensibilité s'accompagne en général d'une mauvaise spécificité. Cette mauvaise spécificité peut engendrer un nombre important de stimulations en faux positifs, et ces sur-stimulations peuvent à leur tour engendrer des réveils intempestifs.

Pour prendre en compte ces aspects, le système de l'invention est capable d'appliquer un traitement qui varie selon le trouble détecté, préférentiellement à l'égard des aspects suivants :
- précocité du traitement : une apnée sera traitée par exemple dans un délai de 5 à 10 s après la détection, afin d'agir au plus tôt ; en revanche, une hypopnée sera traitée par exemple dans un délai de 10 à 15 s après détection, ce qui permet d'éviter d'appliquer une stimulation sur des faux positifs ou sur des hypopnées légères et spontanément réduites ;
- énergie du traitement : lors de la détection d'une apnée, il faut être certain d'appliquer une énergie efficace, donc potentiellement importante ; en revanche, pour une hypopnée, le système pourra commencer la stimulation avec une énergie plus basse, donc avec moins de risque de réveiller le patient, quitte à augmenter cette énergie si le trouble perdure.

Bien qu'une différentiation entre traitements pour l'apnée et l'hypopnée soit proposée dans ce qui précède, on comprendra que ce principe peut s'appliquer à d'autres troubles respiratoires, de types respectifs différentiables : on pourra par exemple différencier le traitement en fonction de la nature de l'apnée (obstructive ou centrale), de la nature de l'hypopnée (obstructive ou centrale), du niveau de la désaturation accompagnant l'épisode, etc.

La Figure 5 illustre schématiquement les différentes actions mises en oeuvre par un système de traitement selon l'invention.

À l'étape 500, le système analyse les signaux fournis par les moyens capteurs 12, 14, 16, 30 (ou seulement l'un d'eux) pour réaliser une différentiation entre apnée (bloc 510) et hypopnée (bloc 520).

Si une apnée est détectée, le système applique une temporisation Δt, par exemple de 6 s (étape 512), avant de déterminer (étape 514) si l'apnée est encore présente. Dans l'affirmative, le traitement est appliqué avec l'énergie nominale adaptée à ce type de trouble - énergie plus élevée que pour une hypopnée (étape 516). Dans la négative, le processus retourne à l'étape 500 de surveillance des signaux.

Si c'est une hypopnée qui est détectée, le système applique à l'étape 522 une temporisation Δt, par exemple de 10 s, puis détermine (étape 524) si le phénomène d'hypopnée est toujours présent. Dans l'affirmative, le système fixe un niveau d'énergie de stimulation minimal (étape 525) et applique un traitement de stimulation 526 avec ce niveau d'énergie. Ensuite, le système détermine à nouveau si l'hypopnée est présente (étape 527). Dans l'affirmative, le niveau d'énergie de stimulation est augmenté d'un incrément donné (étape 528) et le traitement 526 est appliqué avec cette nouvelle énergie, puis il est à nouveau vérifié si l'hypopnée est présente à l'étape 527.

Le test de l'étape 529, mise en oeuvre après chaque incrément d'énergie, permet de limiter le niveau d'énergie à un plafond (typiquement en deçà du niveau d'énergie susceptible de provoquer un réveil du patient), et d'interrompre le traitement lorsque ce niveau d'énergie est atteint.

Dans tous les cas, la stratégie de stimulation elle-même (notamment la durée des impulsions et l'espacement entre elles) est déterminée par le système, dynamiquement, en fonction du type de trouble (central ou obstructif).

Un second aspect du système de l'invention concerne le moment où la stimulation est arrêtée après un retour de la respiration.

Dans l'état général de la technique, il est recommandé d'arrêter la stimulation dès que le signal respiratoire est présent.

Or les inventeurs ont observé qu'un arrêt précoce de la stimulation pouvait dans certaines conditions provoquer des récidives précoces des troubles respiratoires.

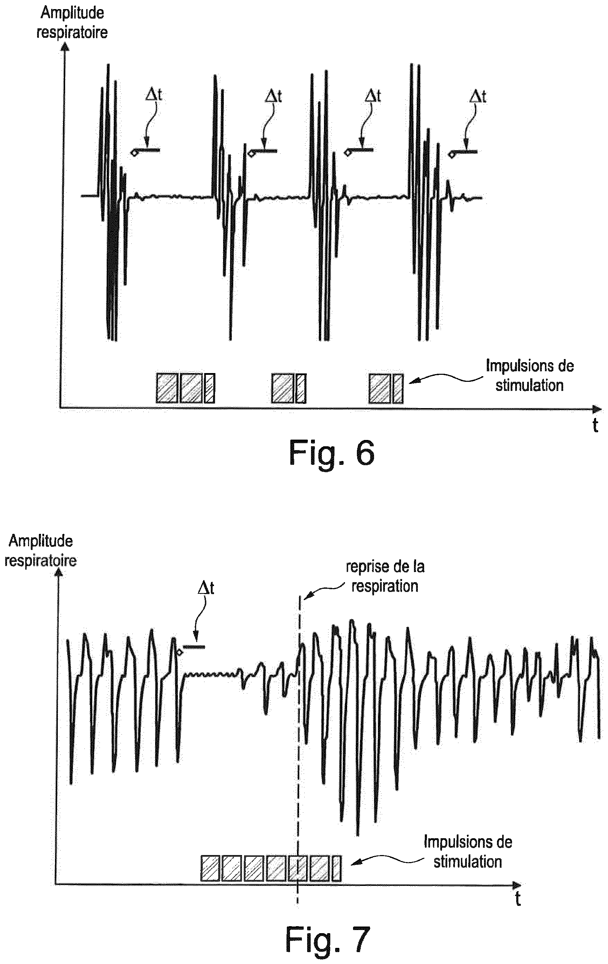

Ainsi, la Figure 6 illustre le cas où des impulsions de stimulation kinesthésique sont appliquées par le module de traitement après un délai déterminé Δt, avec une énergie appropriée. Dès que la reprise respiratoire est observée (reprise de l'amplitude du signal détectant la respiration, comme illustré), le traitement s'arrête au risque de constater comme dans cet exemple un retour précoce du trouble respiratoire.

La Figure 7 illustre quant à elle le principe mis en oeuvre par le système selon ce second aspect de l'invention, selon lequel les impulsions de stimulation kinesthésique sont prolongées au-delà du moment où l'analyse du signal détermine la reprise de la ventilation. On a ainsi constaté que cette prolongation permettait à la ventilation de se rétablir de façon durable, en minimisant le risque de récidive précoce.

L'exemple illustré sur les Figures 6 et 7 est celui des apnées ou hypopnées de type obstructives.

Dans certains cas, il peut être également important d'imposer une limite au nombre d'impulsions délivrées. En effet, au-delà d'un certain nombre, il est fort probable que l'absence de réponse soit due à une inefficacité de la thérapie. Dès lors, il est inutile de continuer à délivrer inutilement des impulsions, qui risquent de réveiller le patient.

Cette limite doit être différente en fonction du traitement délivré. Dans le cas d'un traitement constitué d'impulsions courtes destinées donc à déclencher une réponse de la balance sympathovagale, le réflexe produit est de type "tout-ou-rien". On estime que si les quelques premières impulsions (par exemple les trois premières) délivrées à une amplitude appropriée n'ont pas réussi à déclencher le réflexe, le traitement n'est pas efficace et il est inutile de stimuler plus, au risque de réveiller le patient. Par contre, dans le cas d'un traitement constitué d'impulsions longues destinées à recruter les fibres nerveuses, le nombre maximal d'impulsions est plus important, typiquement entre 10 et 15 impulsions, afin d'être sûr d'avoir recruté le maximum de fibres nerveuses.

La durée additionnelle, ou durée de stabilisation, qui s'écoule entre la détection de la reprise respiratoire et la fin de la stimulation peut être fixe ou programmable, ou encore ajustée dynamiquement en fonction de différents critères. Ainsi, un apprentissage peut être avantageusement prévu pour régler au mieux cette durée, exprimée par exemple par le nombre de cycles respiratoires à observer avant l'arrêt du traitement et/ou en fonction des récidives éventuelles observées au cours du temps.

Dans l'exemple de la Figure 7, le système applique après la reprise de la respiration des impulsions ayant même durée et même espacement que celles qui ont causé la cessation du trouble, et ceci pendant une durée correspondant à trois cycles respiratoires d'amplitude supérieure à un certain seuil.

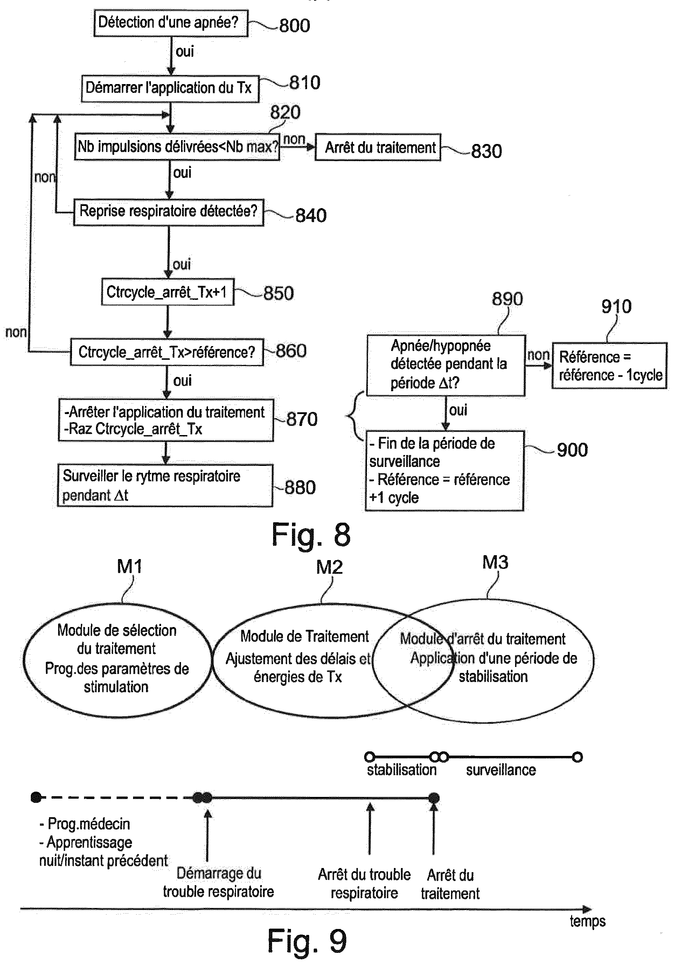

La Figure 8 illustre les différentes étapes mises en oeuvre par le système pour réaliser cette fonction.

L'étape 800 est celle de la détection d'un trouble respiratoire. Lorsqu'un tel trouble est détecté, un certain traitement de stimulation *Tx* est sélectionné en fonction du type de trouble (typiquement apnée/hypopnée, et central/obstructif), et appliqué à l'étape 810.

Pendant l'application du traitement, le système vérifie à l'étape 820 si le nombre d'impulsions délivrées est bien inférieur au nombre maximum alloué pour le type de traitement en cours, par exemple trois impulsions pour un traitement à impulsions courtes (réponse du système sympathovagal) et dix impulsions pour un traitement à impulsions longues (recrutement de fibres). Si ce nombre maximum d'impulsions est atteint, le traitement est arrêté à l'étape 820 même si la reprise ventilatoire n'a pas eu lieu, car on estime que le traitement n'est pas efficace et qu'il convient de ne plus délivrer de stimulations inefficaces, pour éviter de réveiller le patient abusivement.

Ensuite, toujours pendant l'application du traitement *Tx,* le système détecte à l'étape 840 si l'activité respiratoire reprend. Dans l'affirmative, les étapes 850 et 860 réalisent respectivement un comptage du nombre de cycles respiratoires suivant la reprise de la respiration (paramètre *Ctrcycle_arrêt_Tx*) et une comparaison du compte avec une valeur référence qui est le nombre de cycles respiratoires, après reprise, pendant lesquelles la stimulation doit se poursuivre. Lorsque la référence est atteinte, le système arrête l'application des impulsions et remet à zéro la valeur du compte *Ctrcycle_arrêt_Tx* (étape 870).

Ensuite, à l'étape 880, le rythme respiratoire est surveillé pendant une période Δ*T* et un test est effectué à l'étape 890 pour déterminer si un phénomène d'apnée ou d'hypopnée intervient pendant cette période.

Dans l'affirmative, la valeur référence est incrémentée d'une valeur donnée, par exemple d'une unité (étape 900), pour que lors d'une stimulation suivante la phase de stimulation après reprise de la respiration se prolonge pendant une durée plus grande.

Dans la négative, la valeur référence est décrémentée de la même valeur donnée, par exemple d'une unité (étape 910), de telle sorte que lors de la prochaine stimulation, la prolongation de la phase de stimulation après reprise de la respiration se prolonge pendant une durée raccourcie.

Cette diminution de la valeur référence permet d'éviter une dérive systématique du paramètre de durée de prolongation de traitement en cas de reprises fréquentes d'un trouble pendant la surveillance post-traitement. La Figure 9 illustre sur un diagramme temporel l'agencement et l'interaction entre les différentes fonctionnalités décrites dans ce qui précède, à savoir :
- la fonction de sélection d'un traitement de stimulation particulier adapté au type de trouble respiratoire attendu ou détecté ;
- la fonction d'ajustement dynamique des paramètres temporels et d'énergie des impulsions de stimulation appliquées ; et
- la fonction de détermination de l'arrêt de la stimulation contrôlé après la reprise de l'activité respiratoire.

Ces fonctionnalités sont mises en oeuvre respectivement par trois modules matériels ou logiciels spécifiques M1, M2, M3 implémentés dans le boitier 22, étant observé que le module M2 d'ajustement des paramètres de la stimulation et le module de détermination de l'arrêt M3 sont actifs en parallèle pendant la période dite de stabilisation.

## Revendications

1. Un système de traitement d'un trouble respiratoire chez un patient par stimulation kinesthésique, comprenant :
- au moins un capteur parmi divers capteurs ou électrodes (12, 14, 16) ou des moyens de mesure du débit respiratoire (30) ;
- des moyens de commande (18) aptes à produire des signaux de commande de stimulation kinesthésique en réponse à la détection d'un trouble respiratoire ; et
- au moins un effecteur kinesthésique (20) apte à être appliqué sur un site cutané externe du patient, et comprenant un transducteur électro¬mécanique vibrant apte à recevoir les signaux de commande et à délivrer une énergie de stimulation kinesthésique déterminée par lesdits signaux de commande,
**caractérisé en ce que**:
- ledit système est apte à analyser les signaux fournis par au moins un des capteurs (12, 14, 16, 30) pour réaliser une différentiation entre un trouble respiratoire de type apnée et un trouble respiratoire de type hypopnée ; et
- ledit système comprenne des moyens (22) de détermination automatique de traitement, aptes à sélectionner dynamiquement, en fonction d'un type de trouble attendu ou détecté, une stratégie de stimulation parmi un ensemble de stratégies de simulation mémorisées.

2. Le système de la revendication 1, **caractérisé en ce que** lesdites stratégies de stimulation comprennent au moins une stratégie de stimulation par succession d'impulsions de stimulation d'un premier type, et au moins une stratégie de stimulation par succession d'impulsions de stimulation d'un second type,
les impulsions du second type ayant une durée d'impulsion supérieure à celle des impulsions du premier type,
et/ou les impulsions du second type étant espacées entre elles d'un intervalle de durée supérieure à celle des impulsions du premier type.

3. Le système de la revendication 2, **caractérisé en ce que** :
- les impulsions du premier type ont une durée d'impulsion comprise entre 2 et 3 secondes et sont espacées entre elles d'un intervalle d'une durée comprise entre 2 et 3 secondes, et
- les impulsions du second type ont une durée d'impulsion supérieure à celle des impulsions du premier type et sont espacées entre elles d'un intervalle d'une durée supérieure à celle des impulsions du premier type.

4. Le système de la revendication 1, **caractérisé en ce que** les moyens (22) de détermination automatique de traitement comprennent des moyens de plafonnement de la valeur de l'énergie de stimulation à une valeur maximale.

5. Le système de la revendication 1, **caractérisé en ce que** les moyens (22) de détermination automatique de traitement comprennent des moyens de sélection de l'énergie de stimulation et du caractère tardif ou précoce de la thérapie en fonction du type de trouble attendu ou détecté, ladite sélection comprenant soit une stratégie de stimulation à énergie élevée et thérapie précoce (510-516), soit une stratégie de stimulation à énergie moindre et thérapie plus tardive (520-528).

6. Le système de la revendication 1, **caractérisé en ce que** les moyens (22) de détermination automatique de traitement comprennent des moyens de relèvement incrémental du niveau d'énergie à partir de la valeur initiale, aussi longtemps qu'un trouble respiratoire intervenu ne disparaît pas.

7. Le système de la revendication 6, **caractérisé en ce que** les moyens (22) de détermination automatique de traitement comprennent des moyens de comparaison de chaque valeur d'énergie incrémentée à une valeur maximale.

8. Le système de la revendication 7, **caractérisé en ce que** les moyens (22) de détermination automatique de traitement comprennent des moyens de forçage du niveau d'énergie à une valeur supérieure à la valeur maximale admissible, en cas de détection d'un épisode grave de trouble respiratoire.

## Patentansprüche

1. System zur Behandlung einer Atemstörung eines Patienten durch kinästhetische Stimulation, umfassend:
- mindestens einen Sensor unter verschiedenen Sensoren oder Elektroden (12, 14, 16) oder Mittel zur Messung des Atemflusses (30);
- Steuermittel (18), die in der Lage sind, in Antwort auf die Erfassung einer Atemstörung, Signale zur Steuerung einer kinästhetischen Stimulation zu erzeugen; und
- mindestens einen kinästhetischen Effektor (20), der auf eine äußere Hautstelle des Patienten aufgebracht werden kann und einen vibrierenden elektromechanischen Wandler umfasst, der in der Lage ist, die Steuersignale zu empfangen und eine durch die Steuersignale bestimmte kinästhetische Stimulationsenergie auszugeben,
**dadurch gekennzeichnet, dass**:
- das System in der Lage ist, durch mindestens einen der Sensoren (12, 14, 16, 30) bereitgestellte Signale zu analysieren, um zwischen einer Apnoe-artigen Atemstörung und einer Hypopnoe-artigen Atemstörung zu unterscheiden; und
- das System Mittel (22) zur automatischen Bestimmung einer Behandlung umfasst, die in der Lage sind, in Abhängigkeit einer Art der erwarteten oder erfassten Störung, eine Stimulationsstrategie unter einer Reihe von gespeicherten Stimulationsstrategien dynamisch auszuwählen.

2. System nach Anspruch 1, **dadurch gekennzeichnet, dass** die Stimulationsstrategien mindestens eine Stimulationsstrategie anhand einer Folge von Stimulationsimpulsen einer ersten Art und mindestens eine Stimulationsstrategie anhand einer Folge von Stimulationsimpulsen einer zweiten Art umfassen,
die Impulse der zweiten Art eine längere Impulsdauer als die Impulse der ersten Art aufweisen, und/oder die Impulse der zweiten Art durch ein längeres Zeitintervall, als die Impulse der ersten Art getrennt sind.

3. System nach Anspruch 2, **dadurch gekennzeichnet, dass**:
- die Impulse der ersten Art eine Impulsdauer von 2 bis 3 Sekunden aufweisen und durch ein Zeitintervall von 2 bis 3 Sekunden getrennt sind, und
- die Impulse der zweiten Art eine längere Impulsdauer als die Impulse der ersten Art aufweisen und durch ein längeres Zeitintervall, als die Impulse der ersten Art getrennt sind.

4. System nach Anspruch 1, **dadurch gekennzeichnet, dass** die automatischen Behandlungsbestimmungsmittel (22) Mittel zur Begrenzung des Werts der Stimulationsenergie auf einen maximalen Wert umfassen.

5. System nach Anspruch 1, **dadurch gekennzeichnet, dass** die automatischen Behandlungsbestimmungsmittel (22) Mittel zur Auswahl der Stimulationsenergie und des späten oder frühen Zeitpunkts der Behandlung in Abhängigkeit der Art der erwarteten oder erfassten Störung umfassen, wobei die Auswahl entweder eine Stimulationsstrategie mit einer hohen Energie und frühen Behandlung (510-516) oder eine Stimulationsstrategie mit einer niedrigeren Energie und einer späteren Behandlung (520-528) umfasst.

6. System nach Anspruch 1, **dadurch gekennzeichnet, dass** die automatischen Behandlungsbestimmungsmittel (22) Mittel umfassen, um das Energieniveau ab dem Ausgangswert inkrementell zu erhöhen, solange eine stattfindende Atemstörung nicht verschwindet.

7. System nach Anspruch 6, **dadurch gekennzeichnet, dass** die automatischen Behandlungsbestimmungsmittel (22) Mittel zum Vergleich jedes inkrementellen Energiewerts mit einem Maximalwert umfassen.

8. System nach Anspruch 7, **dadurch gekennzeichnet, dass** die automatischen Behandlungsbestimmungsmittel (22) Mittel umfassen, um das Energieniveau auf einen höheren Wert als der zulässige Maximalwert zu zwingen, wenn ein schwere Atemstörung erfasst wird.

## Claims

1. A system for treating a respiratory problem in a patient through kinesthetic stimulation, comprising:
- at least one sensor among various sensors or electrodes (12, 14, 16) or means for measuring the respiratory flow (30);
- control means (18) adapted for generating kinesthetic stimulation control signals in response to the detection of a respiratory problem; and
- at least one kinesthetic effector (20) that can be applied to a patient's external cutaneous site, and comprising a vibrating electromagnetic transducer adapted for receiving the control signals and for outputting a kinesthetic stimulation energy determined by said control signals,
**characterized in that**:
- said system is adapted for analyzing the signals provided by at least one of the sensors (12, 14, 16, 30) to differentiate between an apnea-type respiratory problem and a hypopnea-type respiratory problem; and
- said system comprises means (22) for automatically determining a treatment, that are adapted for dynamically selecting a stimulation strategy from a set of stored stimulation strategies, in accordance with the type of the expected or detected problem.

2. The system according to claim 1, **characterized in that** said stimulation strategies comprise at least one stimulation strategy through a succession of stimulation pulses of a first type, and at least one stimulation strategy through a succession of stimulation pulses of a second type,
the pulses of the second type having a longer pulse duration than the pulses of the first type, and/or the pulses of the second type being spaced by a longer time interval than the pulses of the first type.

3. The system according to claim 2, **characterized in that**:
- the pulses of the first type have a pulse duration between 2 and 3 seconds and are spaced by a time interval of 2 to 3 seconds,
and
- the pulses of the second type have a longer pulse duration than the pulses of the first type and are spaced by a longer time interval than the pulses of the first type.

4. The system according to claim 1, **characterized in that** the means (22) for automatically determining a treatment comprise means for capping the value of the stimulation energy to a maximum value.

5. The system according to claim 1, **characterized in that** the means (22) for automatically determining a treatment comprise means for selecting the stimulation energy and the late or early timing of the therapy depending on the type of expected or detected problem, said selection comprising either a high energy and early therapy stimulation strategy (510-516) or a low energy and late therapy stimulation strategy (520-528).

6. The system according to claim 1, **characterized in that** the means (22) for automatically determining a treatment comprise means for incrementally raising the energy level starting from the initial value as long as an occurring respiratory problem doesn't subside.

7. The system according to claim 6, **characterized in that** the means (22) for automatically determining a treatment comprise means for comparing each incremental energy value with a maximum value.

8. The system according to claim 7, **characterized in that** the means (22) for automatically determining a treatment comprise means for forcing the energy level to a value above the allowed maximum value in case a severe respiratory problem is detected.
